(19) Europäisches Patentamt
European Patent Office
Office européen des brevets

(11) Publication number: 0 313 538
A2

# (12) EUROPEAN PATENT APPLICATION

(21) Application number: 88850355.4

(22) Date of filing: 20.10.88

(51) Int. Cl.4: **C 12 P 21/00**
G 01 N 33/577, G 01 N 33/66,
C 07 H 11/00, C 07 K 9/00

(30) Priority: 22.10.87 SE 8704114

(43) Date of publication of application:
26.04.89 Bulletin 89/17

(84) Designated Contracting States:
AT BE CH DE FR GB IT LI NL SE

(71) Applicant: CARMEDA AB
Nytorpsvägen 10
S-183 53 Täby (SE)

BIOCARB AB
S-223 70 Lund (SE)

(72) Inventor: Pejler, Gunnar
Wallingatan 14B
S-752 27 Uppsala (SE)

Lindahl, Ulf
Torgvägen 7
S-752 46 Uppsala (SE)

Larm, Olle
Nyängsvägen 86
S-161 39 Bromma (SE)

Lundblad, Arne
Bytaregatan 7
S-222 21 Lund (SE)

(74) Representative: Henningsson, Gunnar et al
Bergling & Sundbergh AB Box 7645
S-103 94 Stockholm (SE)

## (54) Monoclonal antibody, a process for its preparation and the use thereof.

(57) A monoclonal antibody which has specificity towards an antigenic determinant of a saccharide having in reducing-terminal position a 2,5-anhydro hexose residue;
a process for the preparation of such monoclonal antibody;
a process for isolating saccharides; and
a process for determining the presence of a saccharide.

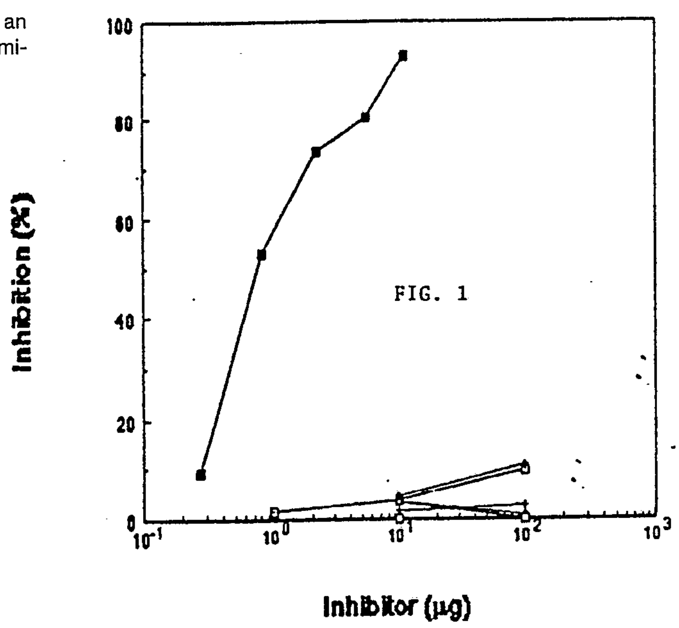


FIG. 1

EP 0 313 538 A2

## Description

### Monoclonal antibody a process for its preparation and the use thereof.

The present invention relates to a monoclonal antibody which has specificity towards an antigenic determinant of certain types of saccharides. Moreover, the invention includes a process for the preparation of such monoclonal antibody and a process for isolating saccharides and determining the presence of saccharides in a sample subject to analysis.

The present invention involves saccharides containing a 2-amino-2-deoxy-glycopyranosyl residue the $NH_2$-function of which in the most stable conformation is equatorially oriented. In raising monoclonial antibodies according to the present invention such saccharide is degraded in respect to the said residue by diazotisation and converted to a terminal 1-deoxy-2,5-anhydrohexitol entity. For details with regard to this known art cf. US patent 4,613,665, the full disclosure of which is incorporated herein by reference thereto. There are mainly four saccharides corresponding to this configuration and these are heparin or heparan sulphate, hyaluronic acid, dermatan sulphate and chitosan. The chemical structure of these four substances will now be discussed in brief.

Heparin

Heparin is built up from alternating glycouronic acid and glucosamine units. The glycuronic acid units consist of D-glycuronic acid and L-iduronic acid. These are respectively β- and α-(1,4)-bound to the D-glucosamine units. A large proportion of the L-iduronic acid residues are sulfated in 2-position. The D-glucosamine units are N-sulfated, sulfated in 6-position and are α-(l,4)-bound to the uronic acid residues. Certain D-glucosamine units are also sulfated in the 3-position .

Hyaluronic acid

Hyaluronic acid is composed of alternating 1,4-bound β-D-glucuronic acid and 1,3-bound N-acetyl-β-D-glucosamine units.

Dermatan sulfate

Dermatan sulfate is composed of alternating L-iduronic acid and N-acetyl-D-galactosamine units which are respectively α-(1,3)- and β-(1,4)-bound. The polysaccharide is partially O-sulfated.

Chitosan (ref. 3)

Chitosan is built up from β-(1,4)-bound D-glucosamine residues.

In connection with experiments, where monoclonal antibodies were raised against a conjugate between oligosaccharides and human serum albumin it was unexpectedly found that these antibodies recognized a determinant present in oligosaccharide deamination products but not in intact saccharide or in a variety of related saccharides. The oligosaccharides tested were prepared by partial nitrous acid degradation of inter alia heparin, and were coupled to lysine residues in human serum albumin by reductive amination. (For details see the above US patent). The only structural element that is present in the deamination products but absent in the intact saccharide is a 2,5-anhydro-D-hexitol residue located at the reducing end of the oligosaccharides. It was therefore concluded that this structure is essential for interaction with the monoclonal antibodies.

The present invention thus provides for a monoclonal antibody which has specificity towards an antigenic determinant of a saccharide having in reducing-terminal position a 2,5-anhydrohexitol residue.

Such antigenic determinant is preferably comprised of not less than four monosaccharide residues including the 2,5-anhydrohexitol residue.

In a particularly preferred embodiment of the invention said determinant comprises, in addition to the 2,5-anhydrohexitol residue, two hexuronic acid residues flanking one hexose amine residue. An example of such determinant is one wherein the 2,5-anhydrohexitol is 2 ,5-anhydro-D-mannitol.

It is preferred that the hexose amine residue has α,1-4-configuration and carries an N-sulphate or N-acetyl group. A particularly preferred saccharide is one derived from heparin or heparan sulphate. Such antigenic determinant preferably has the following structure:

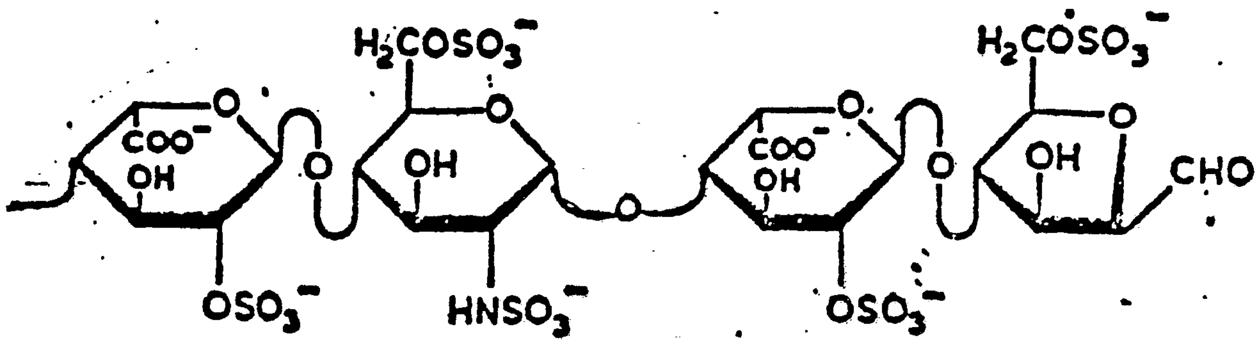

The present invention also provides for a process for preparing a monoclonal antibody which has specificity towards an antigenic determinant of the type referred to above. Such a process comprises the steps:

a) immunizing an animal with such saccharide or a conjugate of such saccharide linked through its free terminal aldehyde group to a macromolecular carrier;

b) sacrificing the animal and recovering its spleen or lymph node cells;

c) fusing said cells with myeloma cells to form hybrid cells;

d) subjecting the hybrid cells resulting from step c) to selection to recover the desired clone;

e) multiplying the selected clone; and

f) allowing said clone to express the monoclonal antibody and recovering same.

In such process the animal is preferably selected from mice, rats and humans. In the process there is preferably used a conjugate of the saccharide and a serum albumin.

The finding of the present invention is useful in several applications based on the interaction between antigenic determinant and monoclonal antibody. Among such applications there may be mentioned a process for the isolation of a saccharide of the type indicated, such process comprising the steps:

a) preparing a carrier having immobilized thereon a monoclonal antibody as defined above;

b) contacting the carrier resulting from step a) with said saccharide;

c) separating the antibody-saccharide complex; and

d) releasing the saccharide from the immobilized monoclonal antibody and recovering same.

Another application involves a process for determining the presence of a saccharide of the type indicated in a sample or other material subject to test, and such process is again based on the interaction between said saccharide or rather its antigenic determinant and a monoclonal antibody according to the invention.

In connection with research and experimentation leading to the present invention it has been surprisingly found that the monoclonal antibodies raised against a conjugate between an oligosaccharide and a serum albumin interacts not only with the antigenic determinant when present in such conjugate but also when present in the saccharide in free form. This fact is, of course, of significance since it means that a monoclonal antibody according to the invention can be used for detection of both a specific oligosaccharide in free form and in the form of a conjugate covalently coupled to for example a substrate or to an albumin. The finding as indicated above thus adds significantly to the versatility of the invention in regard to its practical application.

The invention will now be further illustrated by non-limiting examples with reference to the appended drawings, wherein:

Fig. 1 shows a diagram on inhibition of binding as a function of concentration of inhibitor;

Fig. 2 is a diagram on inhibition as a function of the number of monosaccharide units;

Fig. 3 shows an affinity chromatogram of tetrasaccharides on immobilized monoclonal antibody;

Fig. 4 is a diagram on gel chromatography of products formed by nitrous acid degradation of $^{3}H$-labelled tetrasaccharides;

Fig. 5 shows diagrams on exchange HPLC of $^{3}H$-labelled disaccharides; and

Fig 6. shows the structure of the antigenic determinant recognised by the monoclonal antibody of the invention.

In the present disclosure the abreviations used are:

HSA, human serum albumin;
HPLC, high performane liquid chromatography;
ELISA, enzyme-linked immunosorbent assay;
HexA, unspecified hexuronic acid;
IdoA, L-idopyranosyluronic acid;
GlcA, D-glucopyranosyluronic acid;
GlcNAc, 2-deoxy-2-acetamido-D-glucopyranose (N-acetyl-D-glucosamine);
aMan, 2,5-anhydro-D-mannose;
$aMan_R$, 2,5-anhydro-D-mannitol formed by reduction of terminal 2,5-anhydro-D-mannose residues with $NaBH_4$;
$-NSO_3$, N-sulfate;

$-OSO_3$, O-sulfate, ester sulfate group. The locations of O-sulfate groups are indicated in parenthesis. The various sugar units are connected by 1-4 linkages of the appropriate configuration (α-D-glucosaminidic; β-D-glucuronidic; α-L-iduronidic).

GENERAL BACKGROUND

Heparin is a sulfated glycosaminoglycan consisting of hexuronic acid (D-glucuronic or L-iduronic acid) and glucosamine residues in alternating sequence. The aminosugar can either be N-sulfated or N-acetylated and, in addition, both the hexuronic acid and glucosamine moietys carry O-sulfate groups at various positions (Bienkowski & Conrad, 1985; Ro den, 1980; Lindahl & Kjellen, 1987).

A few reports on antibodies against glycosaminoglycans have occurred. Christner et al (1980) described a polyclonal antiserum that recognized an antigenic determinant present in chondroitinase ABC-digested bovine cartilage proteoglycans. Later Couchman et al (1984) characterized monoclonal antibodies against chondroitin sulfate proteoglycans and defined the antigenic determinants as chondroitin sulfate disaccharide units of various structures. However, exposure of the antigenic structures required digestion with chondroitinase ABC, since the unsaturated uronic acid residues produced by the enzyme treatment were necessary for recognition by the antibodies. Similar monoclonal antibodies against chondroitin sulfate have also been described by Jenkins et al (1981). Monoclonal antibodies reacting with intact keratan sulfate have been described by Caterson et al (1983) and, recently, monoclonal antibodies recognizing intact heparan sulfate (Kure & Yoshie, 1986) and chondroitin sulfate (Yamagata et al, 1987) have been reported.

MATERIALS USED

Unlabeled pig mucosal heparin, chondroitin sulfate, dermatan sulfate, hyaluronan (Höök et al, 1982) and heparan sulfate (preparation HS I in Lane et al., 1986) were as described. Dextran sulfate (1.8 sulfate groups/monosaccharide residue) (Mr 17500) was purchased from Pharmacia, Uppsala, Sweden. Heparin oligosaccharides were obtained by partial deaminative cleavage of the polysaccharide with nitrous acid. To initiate depolymerization, 42 mg of $NaNO_2$ was added to 1 g of pig mucosal heparin in 20 ml of ice-cold $H_2O$, acidified to pH 1.5 with dilute $H_2SO_4$, and the reaction mixture was then kept in an ice bath for 3 hours. Oligosaccharides precipitated with 10 volumes of ethanol were separated by gel chromatography on Sephadex G-50 as described (Lane et al, 1984). Oligosaccharides containing between 2 and 20 monosaccharide units were obtained essentially in homogenous form. $^3H$-labeled disaccharide standards with the general structure HexA- $[^3H]aMan_R$[1)], with O-sulfate groups at various positions were obtained by $HNO_2$(pH 1.5)-$NaB^3H_4$ treatment of unlabeled heparin followed by anion-exchange HPLC (Thunberg et al, 1982). Affigel 10 was purchased from BioRad Laboratories, Richmond, CA; human serum albumin (HSA), grade A from KabiVitrum AB, Stockholm, Sweden; Sephadex G-25 from Pharmacia, Uppsala, Sweden; nitrocellulose filters BA-85 (25 mm), 0.45 µm from Schleicher & Schuell, Dassel, West Germany; and $NaB^3H_4$ (5-15 Ci/mmol) was obtained from New England Nuclear. Monoclonal antibodies against a glucose-containing tetrasaccharide (IgG) and against blood group A (IgM) were used as control immunoglobulins.

EXAMPLE 1

Preparation of Heparin Oligosaccharide-HSA Conjugate.

Heparin oligosaccharides (16-20 monosaccharide units, average Mr ~ 5400), containing reducing-terminal 2.5-anhydro-D-mannose units (with a free aldehyde group), were coupled through reductive amination to (amino groups of) lysine residues in HSA. One hundred mg of HSA and 50 mg of heparin oligosaccharides, prepared by partial nitrous acid depolymerization of heparin were dissolved in 20 ml of 0.1 M acetate buffer, pH 5.2. After addition of 10 mg of $NaBH_3CN$ the reaction mixture was left at room temperature for 48 h (Hoffman et al., 1983). Separation of the conjugate from free albumin and free oligosaccharides was achieved by a slight modification of the procedure described by Hennink et al (1983). The reaction mixture was applied directly to a column (2.5 x 6 cm) of DEAE-cellulose (Whatman, DE 52) equilibrated with 0.05 M acetate buffer, pH 4.0, containing 0.05 M LiCl. Free albumin was removed by washing the column with ~5 volumes of the same acetate buffer and subsequently the column was eluted with ~10 volumes of 0.05 M acetate buffer pH 4.0, containing 3 M LiCl. The recovered material was dialyzed against 0.2 M $NH_4HCO_3$, lyophilized and applied to a column (2.5 x 9 cm) of Cibacron Blue Sepharose (Pharmacia). Free oligosaccharide was removed by washing the column with ~10 volumes of 0.025 M Tris-HCl, containing 0.17 M NaCl and finally the heparin oligosaccharide-HSA conjugate was eluted with -4 volumes of 0.025 M Tris-HCl, pH 7.5 containing 0.17 M NaCl and 0.25 N KSCN. Fractions positive for both protein and uronic acid were pooled, dialyzed extensively against 0.2 M $NH_4HCO_3$ and lyophilized. Quantitative protein and oligosaccharide analysis showed that the conjugate contained HSA and heparin oligosaccharide in a molar ratio of approximatively 1:1.

EXAMPLE 2

Preparation of heparinized ELISA-Plates.

Microtiterplates (96 wells, 86 x 128 mm) from Nunc (Denmark) were aminated with crosslinked polyethyleneimine, essentially as described by Larm et al. (1983). The aminated plates were treated with an aqueous solution of oligosaccharides prepared by partial nitrous acid degradation of heparin (0.25 g/l),

$NaBH_3CN$ (0.025 g/l) and NaCl (8.8 g/l) at pH 3.9 and 50° for two hours (Hoffman et al., 1983). After extensive rinsing with ultrapure water, borate buffer (0.15 M, pH 9.0) and ultrapure water, the presence of covalently bound heparin was confirmed by staining with an aqueous solution of toluidine blue (0.2 g/l) (Larsson et al., 1977).

EXAMPLE 3

Immunization, Fusion and Cloning.

Balb/cABom mice (Gl Bomholtgaard, Ry, Denmark), 8 weeks old, were immunized with the heparin oligosaccharide-HSA conjugate. Fifty micrograms in complete Freund's adjuvant were given subcutaneously on day 1, and on day 14 another 50 µg in incomplete Freund's adjuvant were injected subcutaneously. Fusion was performed on day 37 after final high booster doses (400 µg, intraperitoneal injections) given on days 33, 34, 35 and 36. The fusion and cloning procedures have been described (Messeter et al., 1984). SP 2/0 plasmacytoma cells were mixed with spleen cells from immunized mice in a ratio of 1:2,3.

EXAMPLE 4

ELISA for Antibody-producing Hybridomas.

Supernatants (50 µl) from microtiter wells with growing hybridoma were transferred to heparinized microtiter wells and incubated for 30 min at room temperature. After washing (3 times) with phosphate-buffered saline containing 0.05% Tween 20 (PBS-Tween), 100µl of rabbit anti-mouse immunoglobulin conjugated to horseradish peroxidase (Dakopatts, Copenhagen, Denmark) was added in 1/500 dilution and incubated for 30 min at room temperature. The wells were washed (4 times) with PBS-Tween before adding the enzyme substrate solution containing 0.1% recrystallized 5-amino-salicylate in 0.01 M sodium phosphate buffer, pH 6.0 and $H_2O_2$ freshly added to a concentration of 0.01%. Optical density was read after 30 min at 450 nm.

EXAMPLE 5

Production of ascites.

The established monoclonal cell lines were grown as ascites tumors in Balb/c mice treated with Pristane (Aldrich, Beerse, Belgium). Control ascites was also produced from the non-secreting SP 2/0 plasmacytoma cells.

EXAMPLE 6

Determination of Isotype.

Isotyping of the antibodies was performed by double immunodiffusion in agarose, using rabbit antisera against isotypes of mouse immunoglobulins (Miles, Elkhart, IN, USA).

EXAMPLE 7

Nitrocellulose Filter Assay for Antibody-Antigen Interaction.

Binding of antibodies to $^3H$-labeled heparin oligosaccharides and inhibition by unlabeled oligo- or polysaccharides were determined in a nitrocellulose filter assay, essentially as described (Zopf et al., 1978). Samples (1 µl) of antibody-containing ascites fluid were mixed with 5000 cpm (specific activity 90000 cpm/µg of uronic acid) of $^3H$-labeled heparin oligosaccharides (8-14 monosaccharide units) and varying amounts of unlabeled inhibitors, in a final volume of 500 µl Tris buffer 1 (0.01 M Tris-HCl, pH 7.5 containing 0.14 M NaCl, 0.5 mM $MgSO_4 \times 7H_2O$, 0.15 mM $CaCl_2$ and 0.02% w/v $NaN_3$). All dilutions of inhibitors, antibodies and $^3H$-labeled heparin oligosaccharides were performed in Tris buffer 1. After incubation at 4° C overnight the mixtures were passed through nitrocellulose filters, which were subsequently washed with 10 ml of Tris buffer 1 and finally dissolved in 2 ml of dioxan and counted by liquid scintillation counting. In typical experiments with antibodies and labeled oligosaccharides, 2000-3000 cpm were bound to the filters in the absence of unlabeled saccharide inhibitors. Only 20-30 cpm were unspecifically retained by the nitrocellulose filters in control incubations without added antibody, or whith control IgG/IgM or control ascites fluid substituting for the anti-heparin antibodies.

EXAMPLE 8

Coupling of Antibodies to Affigel 10.

Ascites fluid (750 µl) containing antibodies (539/4H6) was dialyzed against 0.1 M Hepes, pH 7.5, containing 0.1 M NaCl. Affigel 10 (2 ml) was washed with one volume of isopropyl alcohol followed by three volumes of deionized water and was subsequently mixed with the antibody solution. After shaking the mixture for 1 h at room temperature, 0.1 ml of 1 M ethanolamine, pH 8.0, was added and agitation was continued for another 30 min. Finally, the gel was washed with Tris buffer 1 containing 3 M NaCl and subsequently equilibrated with Tris

buffer 1.

EXAMPLE 9

Radiolabeling of Oligosaccharides.

Oligosaccharides recovered after partial nitrous acid deamination of heparin were radiolabeled by reduction of their terminal anhydromannose unit to 2.5-anhydro-D-[$^3$H]mannitol with $NaB^3H_4$. To oligosaccharides (2 mg) dissolved in 0.5 ml 0.5 M $Na_2CO_3$ were added 5 mCi $NaB^3H_4$ (specific activity, 5-15 Ci/mmol) in 200 µl 0.01 M NaOH, and the mixtures were left at room temperature overnight. Following the addition of unlabeled $NaBH_4$ (10 mg), reduction was allowed to proceed for another 3 h. Finally, remaining $NaBH_4$ was decomposed by lowering the pH to ~4 with glacial acetic acid (in a fume hood) and the samples were neutralized with 4 M NaOH. Radiolabeled oligosaccharides (specific acitivity ~-.1-0.3 x $10^6$ cpm/µg of uronic acid) were recovered by passage through a column (1.5 x 150 cm) of Sephadex G-15 equilibrated with 0.2 M $NH_4HCO_3$ and were then desalted by lyolphilization.

Gel chromatography was performed on a column (1 x 190 cm) of Sephadex G-25 (superfine grade) eluted with 0.2 M $NH_4HCO_3$ at ~6ml/h. $^3$H-Labeled disaccharides were separated by anion exchange HPLC on a Partisil-10 SAX column (Whatman) using step concentrations of $KH_2PO_4$ buffers (Bienkowski and Conrad, 1985; Pejler et al, 1987). Cleavage by nitrous acid of tetrasaccharides with the general structure, HexA-GlcNSO$_3$-HexA-aMan, followed by reduction of the resulting disaccharides, was performed as described (Pejler et al., 1987). Uronic acid was determined by the carbazole reaction (Bitter and Muir, 1962).

RESULTS AND DISCUSSION

In the following disclosure reference is made to the drawings, and for the purpose of a better understanding of the contents thereof there is given a brief explanation to the significance of what is illustrated in the drawings.

Fig. 1 Specificity of binding of monoclonal antibodies to heparin oligosaccharides.

$^3$H-Labeled heparin oligosaccharides (~5000 cpm) were mixed with ascites fluid (1 µl) containing monoclonal antibodies (539/4H6) and various amounts of unlabeled dextran sul fate(⊡), heparin (♦), heparan sulfate (▲), chondroitin sulfate (◇), dermatan sulfate (+), hyaluronan (□) or oligosaccharides (containing 20 monosaccharide units) prepared by partial nitrous acid deamination of heparin (■). The amount of radiolabeled antigen bound to the antibodies was quantitated by the nitrocellulose filter assay (see Example 7). The effects of the unlabeled saccharides are expressed as % inhibition of the binding of radiolabeled antigen obtained in the absence of competitive ligands.

Fig. 2 Size of the antigenic determinant.

$^3$H-Labeled heparin oligosaccharides (~5000 cpm) were mixed with ascites fluid (1 µl) containing monoclonal antibodies (539/4H6) and a 70-fold molar excess of unlabeled heparin oligosaccharides of various chain length. The amount of radiolabeled antigen bound to the antibodies was quantitated by the nitrocellulose filter assay (see Example 7). Results are expressed as in Fig. 1.

Fig. 3 Affinity chromatography of tetrasaccharides on immobilized monoclonal antibodies.

Monoclonal antibodies (539/4H6) against heparin oligosaccharides were covalently attached to Affi-Gel 10 (see Example 10). Tetrasaccharides prepared by partial nitrous acid depolymerization of heparin were radiolabeled by reduction with $NaB^3H_4$ (see Example 9) and ~2 x $10^6$ cpm were applied to a column (1 x 3 cm) of the affinity matrix equilibrated with Tris buffer 1. Unbound material was washed away with ~15 volumes of Tris buffer 1 and subsequently the column was eluted with ~10 volumes of Tris buffer 1 containing 3 M NaCl. No radioactivity remained bound to the column after elution with the latter buffer. Fractions (~5 ml) were collected and analyzed for radioactivity.

Fig. 4 Gel chromatography of products formed by nitrous acid degradation of $^3$H-labeled tetrasaccharides.

[1-$^3$H]aMan$_R$-labeled tetrasaccharides with (□) or without (■) affinity for the monoclonal antibody, 539/4H6, were subjected to complete nitrous acid (pH 1.5) degradation and samples (~0.5 x $10^6$ cpm) were applied to a column (1 x 190 cm) of Sephadex G-25 equilibrated with 0.2 M $NH_4NCO_3$. Fractions (~2ml) were collected and analyzed for radioactivity. The elution positions of standard tetrasaccharides (IdoA-GlcNAc(6-OSO$_3$)-GlcA-aMan$_R$(3,6-di-OSO$_3$)) and disaccharides (IdoA(2-OSO$_3$)-aMan$_R$(6-OSO$_3$)) are indicated by arrows. Fractions containing disaccarides were pooled (as indicated by the horizontal bar), lyophilized, and analyzed further by anion-exchange HPLC, either directly or after reduction with $NaB^3H_4$ (Fig. 5).

Fig. 5 Anion exchange HPLC of $^3$H-labeled disaccharides.

[1-$^3$H]aMan$_R$-labeled tetrasaccharides with (panels A and C) or without (panels B and D) affinity for the monoclonal antibody, 539/4H6, were cleaved by treatment with nitrous acid (pH 1.5), and the resulting labeled disaccharides, representing the reducing-terminal half of the molecules, were recovered (Fig. 4) and

subjected to anionexchange HPLC (panels A and B). Alternatively, the resulting disaccharide mixtures were again reduced with $NaB^3H_4$ (of the same specific activity as that employed in the initial reduction of tetrasaccharides), and the products, now representing the total disaccharide units of the tetrasaccarides, were analyzed in a similar fashion (panels C and D). The analyses were conducted on a SAX Partisil 10 column eluted with $KH_2PO_4$ of the concentrations indicated (---, stepwise concentration increments). The elution positions of standard disaccharides are indicated by arrows: 1, GlcA(2OSO3)-aMan$_R$ (tentatively identified; see Bienkowski and Conrad, 1985); 2, GlcA-aMan$_R$(6-OSO$_3$); 3, IdoA-aMan$_R$(6-OSO$_3$); 4, IdoA(2-OSO$_3$)-aMan$_R$; 5, IdoA(2-OSO$_3$)-aMan$_R$(6-OSO$_3$).

Fig. 6 Structure of the antigenic determinant recognized by the monoclonal antibody 539/4H6.
Specificity of Binding. Production of monoclonal antibodies against the heparin oligosaccharide-HSA conjugate yielded four stabile hybridoma clones that secreted antibodies with strong reactivity against the heparinized ELISA plates. Three of the clones produced IgG antibodies (539/5D3, 538/IF4 and 539/5D4) and one produced antibodies of the IgM class (539/4H6). The specificity of the binding was tested in a nitrocellulose filter assay (see Example 7). A mixture of $^3H$-labeled heparin oligosaccharides containing between 8 and 14 monosaccharide units was used as antigen, and various unlabeled glycosaminoglycans or oligosaccharides were tested as competitive ligands (Fig. 1). The monoclonal antibody 539/4H6 showed no reactivity against intact heparin, heparan sulfate, dextran sulfate, chondroitin sulfate, dermatan sulfate or hyaluronan. In contrast, strong reactivity with a heparin oligosaccharide of 20 monosaccharide units (Mr ~6000) was observed (Fig. 1). Similar results were obtained for the three other monoclonal antibodies (not shown). The heparin oligosaccharide used was prepared by partial nitrous acid deamination and therefore contains, at its reducing end, an anhydromannose residue derived from an N-sulfated glucosamine unit of the intact polysaccharide. Since this component represents the only structural difference between the oligosaccharides, which bound to the antibodies, and the parent heparin chain, which did not bind, these findings strongly indicate that the anhydromannose residue is essential for antibody recognition.

Size of Antigenic Determinant.
To determine the size of the antigenic determinant, the reactivity of the monoclonal antibody 539/4H6 against heparin oligosaccharides of various lengths was tested. The smallest oligosaccharides capable of producing significant inhibition of the binding of radiolabeled antigen was found to be a tet rasaccharide fraction (Fig. 2). As the oligosaccharides used in this experiment were prepared by partial nitrous acid deamination of heparin they all contain anhydromannose at their reducing end. The finding that disaccharides, although they contain the anhydromannose reducing end group, were unable to compete for the binding of radiolabeled antigen, suggests that additional structural elements besides the anhydromannose unit are required for binding to the monoclonal antibodies.

Structure of the Antigenic Determinant.
Nitrous acid deamination of heparin (pH 1.5) converts N-sulfated glucosamine units to 2,5-anhydro-D-mannose residues along with cleavage of the corresponding glucosaminidic bond (Shively & Conrad, 1976). N-acetylated glucosamine residues are resistant to the treatment. The tetrasaccharide fraction used in the present study was prepared by partial nitrous acid deamination and would thus represent a mixture of saccharides with the general structure HexA-GlcNSO$_3$-HexA-aMan or HexA-GlcNAc-GlcA-aMan[2]) with O-sulfate groups at various positions (Shively & Conrad, 1976; Thunberg et al., 1982; Bienkowski and Conrad, 1985).

To further investigate the structural requirements for binding of the monoclonal antibody to the heparin oligosaccharides, an attempt to fractionate the tetrasaccharide mixture into binding and non-binding species was made. Tetrasaccharides were radiolabeled by reduction of the terminal anhydromannose unit with $NaB^3H_4$, and subjected to affinity chromatography on a column of monoclonal antibodies (539/4H6) covalently bound to Affi-Gel 10 (see Example 7). Approximately 50% of the labeled tetrasaccharides bound to the affinity matrix (Fig. 3). Separate rechromatography of the bound and non-bound tetrasaccharide fractions on the same affinity column showed that the initially non-bound fraction contained no binding species and, conversely, that the bound fraction was devoid of non-binding tetrasaccharides (not shown). Samp les (~10000 cpm) of the binding and non-binding tetrasaccharides, respectively, were mixed with soluble antibody (1 µl ascites fluid) and analyzed in the nitrocellulose filter assay. About half (~5000 cpm) of the binding tetrasaccharides compared to only background levels (~20 cpm) of the non-binding tetrasaccharides were retained by the nitrocellulose filters (not shown), further demonstrating the presence of two distinct tetrasaccharide populations containing binding and non-binding species, respectively.

The structures of the binding and non-binding tetrasaccharides were investigated as follows. Each radiolabeled tetrasaccharide fraction was treated with nitrous acid at pH 1.5 and the products were chromatographed on a column of Sephadex G-25 (Fig. 4). Deamination of the binding fraction resulted in complete degradation to disaccharides, demonstrating that the internal glucosamine residue was exclusively N-sulfated. In contrast, nitrous acid treatment of the non-binding tetrasaccharides yielded two peaks corresponding to di- and tetrasaccharides, respectively. The tetrasaccharides resistant to deamination would thus represent structural variants containing an internal N-acetylated glucoseamine residue.

The $^3H$-labeled disaccharides obtained, corresponding to the reducing-terminal half of the intact tetrasaccharides, were recovered and subjected to anion exchange HPLC (Fig. 5). The labeled disaccharides recovered from the tetrasaccharides that bound to the monoclonal antibodies showed a single peak corresponding to IdoA(2-$OSO_3$)-aMan$_R$(6-$OSO_3$) (Fig. 5A). In contrast, similar analysis of disaccharides obtained from the non-binding tetrasaccharides showed a major peak corresponding to GlcA-aMan$_R$(6-$OSO_3$) and, in addition, smaller peaks of GlcA(2-$OSO_3$)-aMan$_R$ (see the legend to Fig. 5), IdoA-aMan$_R$(6-$OSO_3$), IdoA(2-OSO3)-aMan$_R$ and IdoA(2-$OSO_3$)-aMan$_R$(6-$OSO_3$)(Fig 5B).

The nonreducing-terminal disaccharide units of the tetrasaccharide fractions were identified in an indirect fa shion by deaminative cleavage of the (previously endgroup-labeled) tetrasaccharides, followed by reduction of the newly formed anhydromannose residue with $NaB^3H_4$. The resulting mixtures of $^3H$-labeled disaccharides should represent both the reducing- and the nonreducing-terminal disaccharide units of the original tetrasaccharides, in approximately equimolar proportion; ion-exchange chromatograms of these mixtures (Figs. 5C and D) should visualize the latter components, along with those generated on cleavage of merely endgroup-labeled tetrasaccharides (Figs. 5A and B). Such a chromatogram, representing the total disaccharide units derived from the antibody-binding tetrasaccharide (Fig. 5C), showed only a single peak of IdoA(2-$OSO_3$)-aMan$_R$(6-$OSO_3$), similar to the corresponding analysis of the reducing-terminal disaccharide (Fig. 5A). Since a HexA-aMan$_R$ disaccharide derived from the nonreducing end of the intact tetrasaccharide would correspond to a HexA-GlcN$SO_3$-sequence, the overall structure of the tetrasaccharide would be IdoA(2-$OSO_3$)-GlcN$SO_3$(6-$OSO_3$)-IdoA(2-$OSO_3$)-aMan$_R$(6-$OSO_3$). The carbohydrate sequence recognized by antibody 539/4H6 thus is based on the predominant disaccharide unit of heparin (Jacobsson et al., 1979; Bienkowski and Conrad, 1985), and hence is likely to be formed on random, partial deaminative cleavage of the polysaccharide. The antigenic structures recognized by the three other monoclonal antibodies (539/5D3, 538/IF4, 539/5D4) have not yet been identified, but apparently also include the anhydromannose end group.

Similar analysis of the tetrasaccharide fraction lacking affinity for the antibody clone, 539/4H6, indicated the IdoA(2-$OSO_3$)-GlcN$SO_3$(6-$OSO_3$)-sequence as the most frequent structure at the non-reducing end (compare the chromatograms in Fig. 5B and D). It is noted that this sequence also constitutes the non-reducing terminus of the antibody-binding tetrasaccharide; apparently, substitution of structure such as -GlcA-aMan$_R$(6-$OSO_3$) (i.e. the predominant "reducing" terminus of the nonbinding tetrasaccharides; see Fig. 5B) for the -IdoA(2-$OSO_3$)-aMan$_R$(6-$OSO_3$) component of the binding species will abolish the interaction. Conversely, the occurrence of the latter sequence also in the non-binding tetrasaccharide fraction (Fig. 5B) conforms to the notion that this structure alone is not sufficient for recognition by the antibody. The structural requirements for antibody binding are obviously highly stringent.

The present invention finds various applicability, and for example low-molecular weight heparins, prepared by limited deaminative cleavage of the conventional polysaccharide, are of considerable current interest in clinical practice, as an antithrombotic agent with improved properties (Holmer et al., 1986). Recent experiments, to be described elsewhere, have led to a specific and convenient ELISA assay for the determination of such compounds in blood plasma. Further, the antibodies are potentially capable of descriminating between the two fragments generated by a single deaminative cleavage of a heparin chain with nitrous acid, and may thus prove useful in attempts to determine the distribution, within the intact molecule, of specific saccharide sequences. Finally, we may envisage how a panel of such antibodies, of different and defined specificities, may be used in conjugation with size-separation (for instance, by gel electrophoresis; see Rice et al., 1987) of oligosaccharides obtained by limited deaminative cleavage of heparin (or heparan sulfate), to yield detailed sequence information pertaining to the intact polysaccharide chains.

REFERENCES

Bienkowski, M.J. and Conrad, H.E. (1985) J.Bio.Chem.260, 356-365
Bitter, T. and Muir, H.M. (1962) Anal. Biochem. 4, 330-334
Caterson, B., Christner, J.E. and Baker, J.R. (1983) J. Biol.Chem. 258, 8848-8854
Christner, J.E., Caterson. B and Baker, J.R. (1980) J.Biol.Chem. 255, 7102-7105
Couchman, J.R., Caterson, B., Christner, J.E. and Baker, J.R. (1984) Nature 307, 650-652
Hennink, W.E., Feijen, J., Ebert, C.D. and Kim, S.W. (1983) Thromb.Res. 29, 1-13
Hoffman, J., Larm, O. and Scholander, E. (1983) Carbohydr.Res. 117, 328-331.
Holmer, E., Söderberg, K., Bergqvist, D. and Lindahl, U. (1986) Haemostatis 16: suppl. 2, 1-7.
Höök, M., Riesenfeld, J. and Lindahl, U. (1982) Anal. Biochem. 119, 236-245.
Jacobsson, I., Lindahl, U., Jensen, J.W., Roden, L., Prihar, H. and Feingold, D.S. (1984) J.Biol.Chem. 259, 1056-1063.
Jacobsson, I., Höök, M., Pettersson, I., Lindahl, U., Larm, O., Wirén, E. and Von Figura, K. (1979) Biochem.J. 179, 77-87.
Jenkins, R.B., Hall, T. and Dorfman, A. (1981) J.Biol.Chem. 256, 8279-8282.
Kure, S. and Yoshie, O. (1986) J. Immunol. 137, 3900-3908.
Lane, D.A., Denton, J., Flynn, A.M., Thunberg, L. and Lindahl, U. (1984) Biochem.J. 218, 725-732.
Lane, D.A., Pejler, G., Flynn, A.M., Thompson, E.A. and Lindahl, U. (1986) J.Biol.Chem. 261, 3980-3986.
Larm, O., Larsson, R. and Olsson, P. (1983) Med.Dev.Art.Org. 11, 161-173.
Larsson, R., Hjelte, M., Eriksson, J.C., Lagergren, H.R. and Olsson, O. (1977) Trhomb.Hemost. 37, 262-273.
Lindahl, U. and Kjellen (1987) in The Biology of the Extracellular Matrix; Proteoglycans (Wight, T.N. and

Mecham, R., eds), pp 59-104, Academic Press, New York.
Messeter, L., Brodin, T., Chester, M.A., Karlsson, K.A., Zopf, D. and Lundblad, A. (1984) Vox Sang. 46, 66-74.
Pejler, G., Bäckström, G., Lindahl, U., Paulsson, M., Dziadek, M., Fujiwara, S. and Timpl, R. (1987) J.Biol.Chem. 262, 5036-5043.
Rice, K.G., Rottink, M.K. and Linhart, R.J. (1987) Biochem.J. 244, 515-522.
Roden, L. (1980) in The Biochemistry of Glycoproteins and Proteoglycans (Lennarz, W.J., ed.), pp 267-371, Plenum, New York.
Shively, J.E> and Conrad, H.E. (1976) Biochemistry 15, 3932-3942.
Thunberg, L., Bäckström, G. and Lindahl, U. (1982) Carbhydr.Res. 100, 393-410.
Yamagata, M., Kimata, K., Oike, Y., Tani, K., Maeda, N., Yoshida, K., Shimomura, Y., Yoneda, M. and Suzuki, S. (1987) J.Biol.Chem. 262, 4146-4152.
Zopf, D.A., Tsai, C.M. and Ginsburg, V. (1978) Arch.Biochem.Biophys. 185, 61.

## Claims

1. A monoclonal antibody which has specificity towards an antigenic determinant of a saccharide having in reducing-terminal position a 2,5-anhydro hexose residue.

2. A monoclonal antibody according to claim 1, wherein the antigenic determinant is comprised of not less than four monosaccharide residues including the 2,5-anhydro residue.

3. A monoclonal antibody according to claim 2, wherein said determinant in addition to the 2,5-anhydro hexose residue is comprised of two hexuronic acid residues flanking one hexose amine residue.

4. A monoclonal antibody according to any preceding claim 1, wherein the 2,5-anhydro hexose is 2,5-anhydro-D-mannose.

5. A monoclonal antibody according to claim 4, wherein the hexose amine residue has α,1-4-configuration and carries an N-sulphate or N-acetyl group.

6. A monoclonal antibody according to claim 5, wherein the saccharide is derived from heparin or heparan sulphate.

7. A monoclonal antibody according to claim 6, which has specificity towards an antigenic determinant having the following structure:

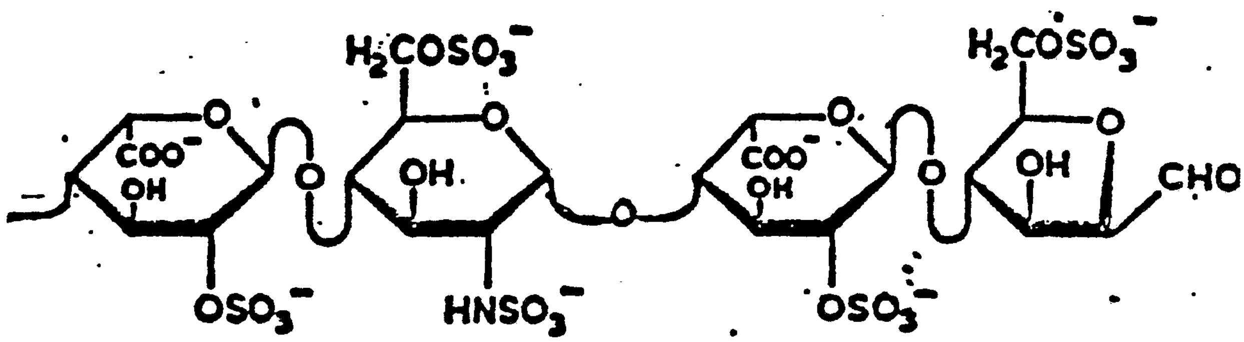

8. A process for preparing a monoclonal antibody which has specificity towards an antigenic determinant of a saccharide having in reducing-terminal position a 2,5-anhydro hexose residue, comprising the steps:

a) immunizing an animal with such saccharide or a conjugate of such saccharide linked through its free terminal aldehyde group to a macromolecular carrier;
b) sacrificing the animal and recovering its spleen or lymph node cells;
c) fusing said cells with myeloma cells to form hybrid cells;
d) subjecting the hybrid cells resulting from step c) to selection to recover the desired clone;
e) multiplying the selected clone; and
f) allowing said clone to express the monoclonal antibody and recovering same.

9. A process according to claim 8, wherein the animal is selected from mice, rats and humans.

10. A process according to claim 8 and 9, wherein there is used a conjugate of the saccharide and a serum albumin.

11. A process for isolating a saccharide having in reducing-terminal position a 2,5-anhydro hexose residue, comprising the steps:

a) preparing a carrier having immobilized thereon a monoclonal antibody according to claim 1;

b) contacting the carrier resulting from step a) with said saccharide;
c) separating the antibody-saccharide complex; and
d) releasing the saccharide from the immobilized monoclonal antibody and recovering same.

12. A process for determining the presence of a saccharide having in reducing-terminal position a 2,5-anhydro hexose residue, wherein there is used the interaction between said saccharide and the monoclonal antibody according to claim 1.

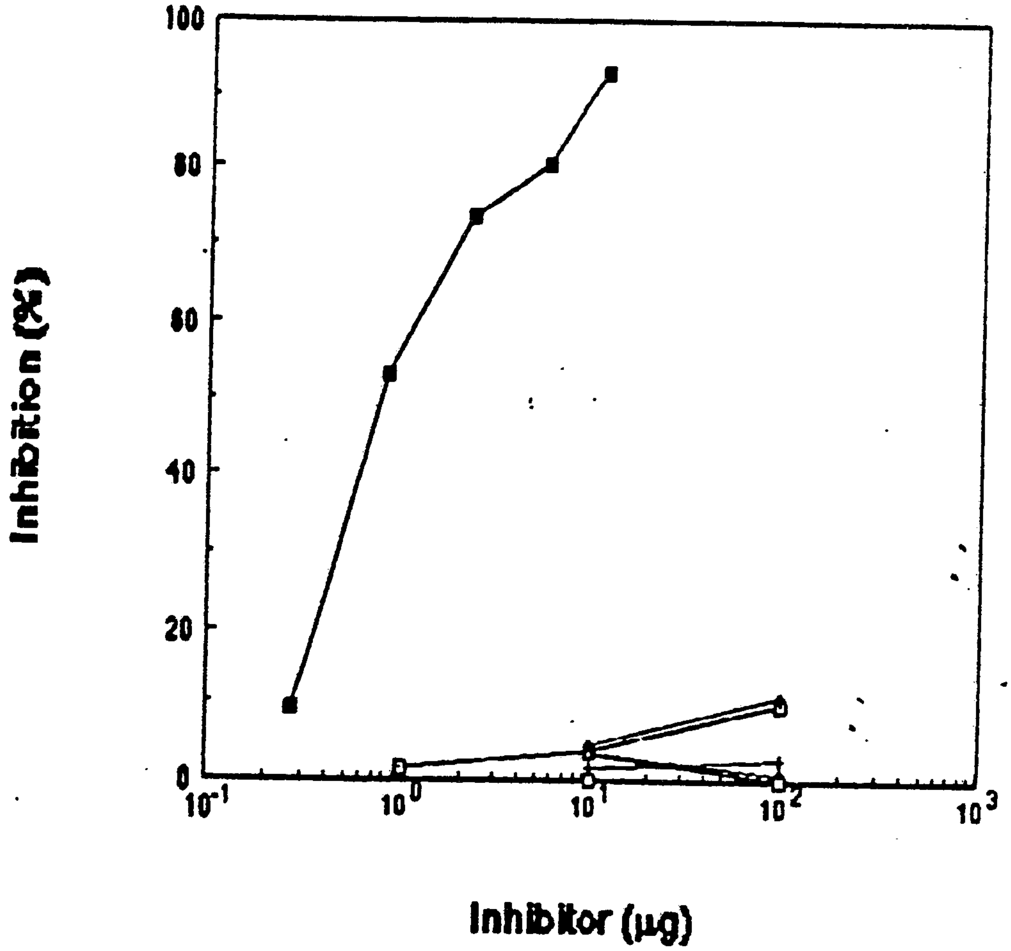

Inhibition (%)
100
80
60
40
20
0
10^-1
10^0
10^1
10^2
10^3
Inhibitor (μg)


FIG. 1

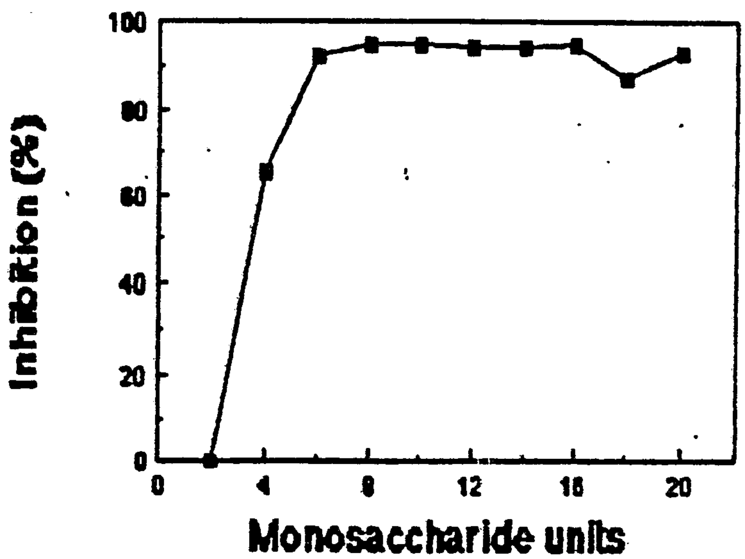

Inhibition (%)
100
80
60
40
20
0
0
4
8
12
16
20
Monosaccharide units


FIG. 2

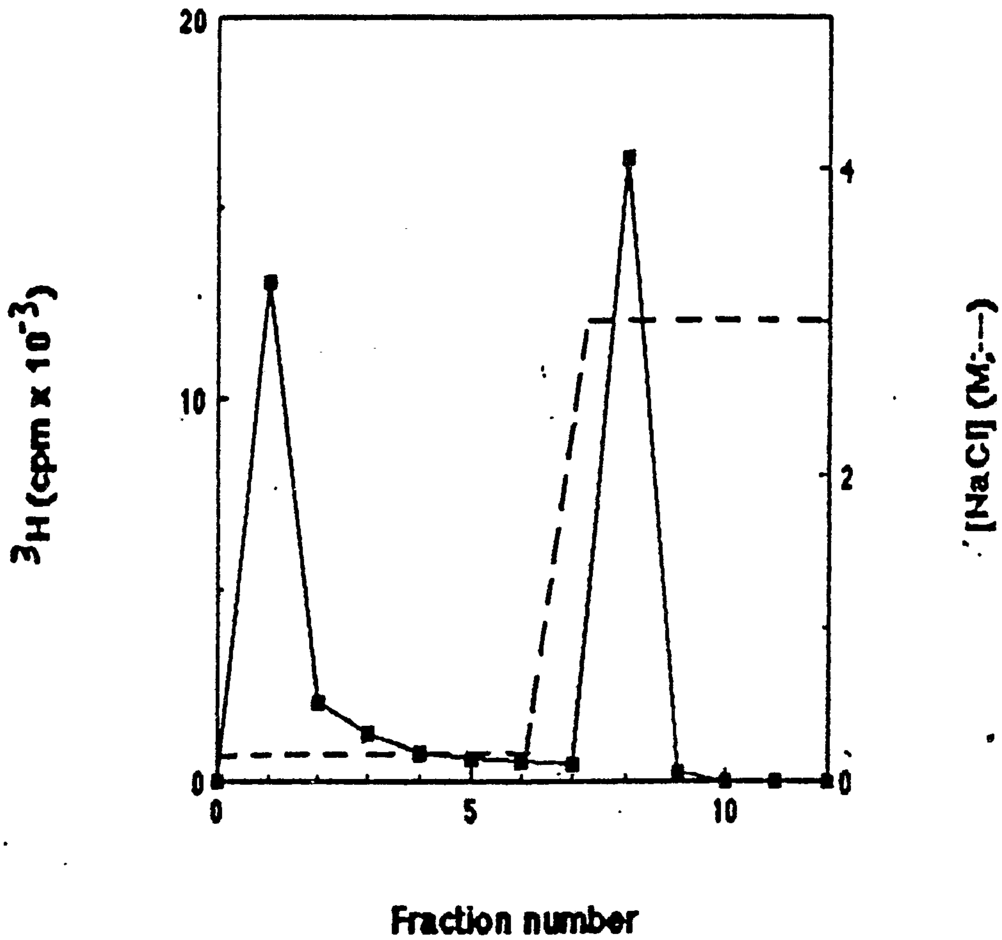
20
10
0
4
2
0
0
5
10
$^3$H (cpm x $10^{-3}$)
[NaCl] (M;---)
Fraction number

FIG. 3

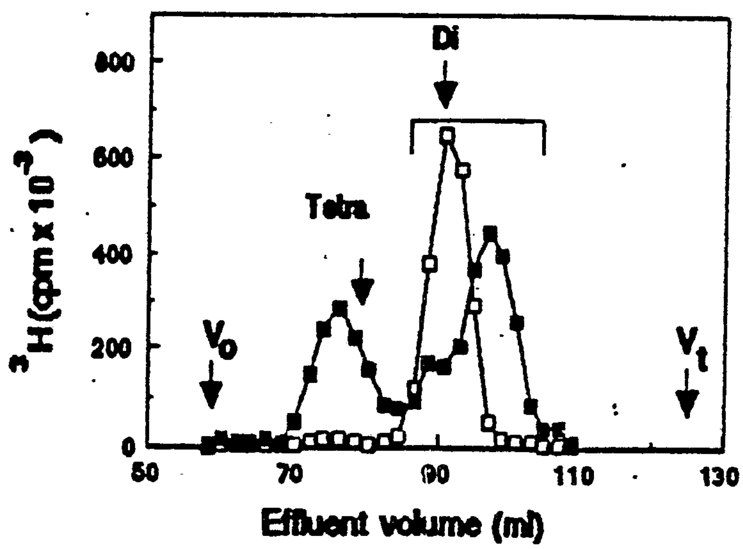
Di
Tetra
$V_o$
$V_t$
800
600
400
200
0
50
70
90
110
130
$^3$H (cpm x $10^{-3}$)
Effluent volume (ml)

FIG. 4

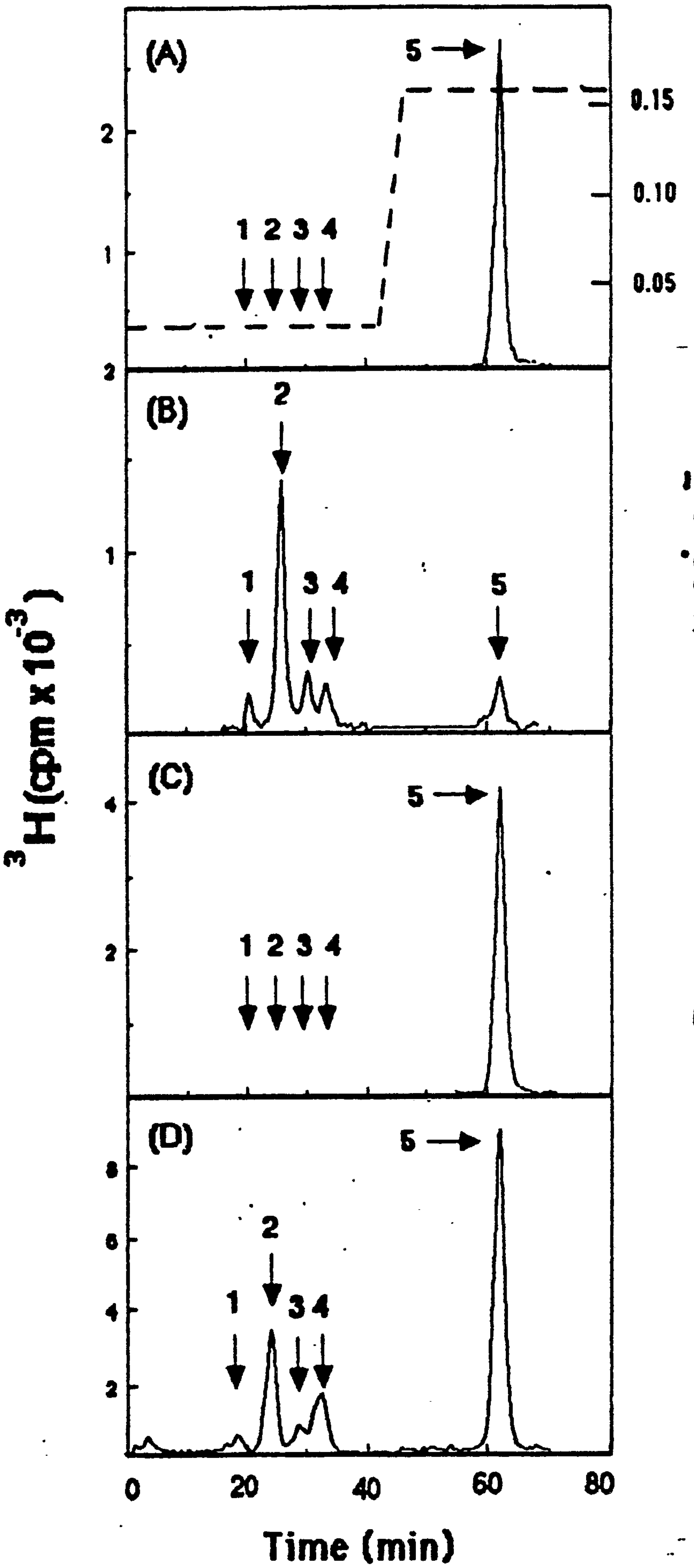
(A)
(B)
(C)
(D)
5
1 2 3 4
0.15
0.10
0.05
[KH2PO4] (M)
3H (cpm x 10-3)
0
20
40
60
80
Time (min)

FIG. 5

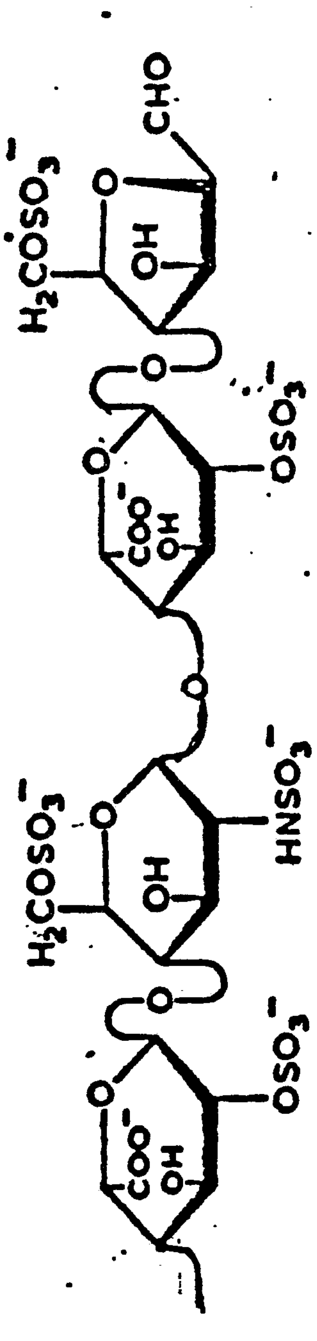

FIG. 6